(19) Europäisches Patentamt
European Patent Office
Office européen des brevets

(11) Numéro de publication: **0 256 933 B1**

(12) # FASCICULE DE BREVET EUROPEEN

(45) Date de publication de fascicule du brevet: **13.05.92**

(51) Int. Cl.5: **A61K 31/235**, A61K 9/54

(21) Numéro de dépôt: **87401824.5**

(22) Date de dépôt: **06.08.87**

(54) **Procédé de préparation d'un médicament à base de fénofibrate, médicament obtenu par ce procédé.**

(30) Priorité: **08.08.86 FR 8611540**

(43) Date de publication de la demande:
**24.02.88 Bulletin 88/08**

(45) Mention de la délivrance du brevet:
**13.05.92 Bulletin 92/20**

(84) Etats contractants désignés:
**AT BE CH DE ES GB GR IT LI LU NL SE**

(56) Documents cités:
**EP-A- 0 145 558**
**WO-A-82/01649**
**DE-B- 1 094 929**
**LU-A- 84 526**

(73) Titulaire: **ETHYPHARM, Société Anonyme**
**21 rue Saint-Mathieu Zone Industrielle**
**F-78550 Houdan(FR)**

(72) Inventeur: **Boyer, Jean-François**
**73 rue des Jeux de Billes**
**F-78550 Houdan(FR)**

(74) Mandataire: **Pinguet, André**
**Cabinet de Proprieté Industrielle CAPRI 28 bis, avenue Mozart**
**F-75016 Paris(FR)**

**EP 0 256 933 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet un nouveau procédé de préparation d'un médicament à base de fénofibrate, ainsi que le médicament obtenu par ce procédé.

On rappelle que le fénofibrate est le para-(4-chlorobenzoyl)-phénoxyisobutyrate d'isopropyle. Dans la présente demande, on désigne par l'expression "fénofibrate et ses dérivés" les composés de formule I

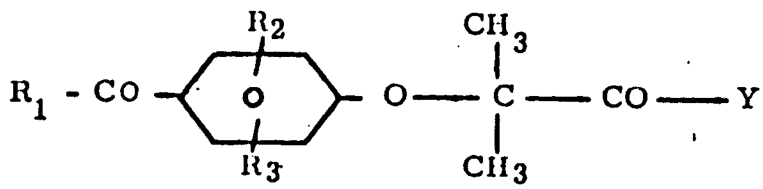

(I)

dans laquelle $R_1$ représente un groupement phényle ou un groupement phényle substitué par un ou plusieurs - $CH_3$, $CF_3$ ou halogènes (notamment fluor, chlore ou brome),

$R_2$ et $R_3$ représentent indépendamment un atome d'hydrogène ou d'halogène (de préférence fluor, chlore ou brome), un groupement alkyle ou alkoxy ayant 1 à 5 C ou un groupement $-CF_3$, $-SCH_3$, $-SOCH_3$, $-SO_2CH_3$ ou -OH, et

Y représente un groupement -OH, un groupement alkoxy inférieur, de préférence en $C_1$ - $C_4$, un groupement $-NR_4R_5$, un groupement $-NHCH_2$ $CH_2$ N $R_4R_5$ ou un groupement -O-alkylène-$NR_4R_5$, l'alkylène ayant notamment 2 à 6 atomes de carbone $R_4$ et $R_5$, identiques ou différents, représentant chacun un atome d'hydrogène, un groupement alkyl en $C_1$ - $C_5$, un groupement cycloalkyle en $C_3$ - $C_7$, de préférence en $C_5$ - $C_6$, un groupe aryle ou aryle substitué sur le reste aromatique par un ou plusieurs groupements halogènes, méthyle ou $-CF_3$,
ou bien $R_4$ et $R_5$ forment ensemble avec l'atome d'azote auquel ils sont reliés, soit un groupe n-hétérocyclique ayant 5 à 7 sommets pouvant renfermer un second hétéroatome choisi parmi N, O et S, et pouvant être substitué, soit un reste amide dérivé de la lysine ou de cystéine.

Bien entendu, l'expression fénofibrate et ses dérivés" englobe en outre les sels d'addition d'acides pharmaceutiquement acceptables, susceptibles d'être obtenus à partir des composés de formule I.

On sait que le fénofibrate est utilisé pour le traitement des hyperlipidémies, hypercholestérolémies et hypertriglycéridémies endogènes de l'adulte. Ainsi, on peut observer chez l'homme soumis à un traitement par le fénofibrate, à raison de 300 à 400 mg par jour une réduction de la cholestérolémie de 20 à 25% et une réduction de la triglycéridémie de 40 à 50%.

Le produit inchangé n'est pas retrouvé au niveau plasmatique. Le métabolite majeur plasmatique est l'acide fénofibrique.

La concentration plasmatique maximale est atteinte en moyenne cinq heures après l'ingestion du médicament. La concentration plasmatique moyenne est de l'ordre de 15 microgrammes/ml pour une posologie de 300 mg de fénofibrate par jour. Ce taux est stable tout au long des traitements continus.

L'acide fénofibrique est fortement lié à l'albumine plasmatique et peut déplacer les antivitamines K des sites de fixation protéiniques et potentialiser leur effet anticoagulant.

La demi-vie plasmatique d'élimination de l'acide fénofibrique est de l'ordre de 20 heures.

Dans ces conditions, on comprend qu'une seule absorption par jour est suffisante.

C'est ainsi qu'une demande de brevet PCT publiée sous le numéro WO 82/01649 s'est déjà préoccupée dans le passé de réduire, avec le dosage, le nombre d'administrations du fénofibrate grâce à une nouvelle forme du médicament correspondant. Pour cela, elle a recours à des granules à base de fénofibrate qui assurent une libération progressive et retardée du produit actif dans l'appareil digestif du patient. Ces granules sont constituées chacune par un noyau neutre entouré d'une première couche où le fénofibrate est présent dans un excipient, cette première couche étant elle-même revêtue d'une seconde couche de protection.

Cependant, si cette forme aboutit à un bon étalement dans le temps du taux plasmatique même dans le cas d'une seule prise quotidienne, ce résultat est avant tout global. On a en effet observé que le fénofibrate avait une mauvaise solubilité dans les liquides aqueux. Pour le patient, il s'ensuit une absorption inégale, notamment dans le tube digestif.

Afin de favoriser cette absorption, il est connu de présenter le produit actif à dissoudre en une poudre dont les particules sont très fines. Elles permettent en effet d'augmenter de façon considérable la surface offerte au processus de dissolution. Par exemple, la demande de brevet allemand DE-B-1 094 929 déposée dès 1957 fait état de cette présentation avantageuse en relation avec bon nombre de produits actifs. Sans

pour autant stipuler parmi eux le fénofibrate, elle indique qu'on la réalise en particulier grâce à des granules de constitution analogue à celles évoquées ci-dessus. Ces dernières comportent alors une première couche où les particules de produit actif sont noyées au sein d'un liant soluble notamment dans l'eau. Toutefois il est prévu que les particules aient des dimensions comprises entre 5 et 200 µm environ.

Pour ce qui est du fénofibrate, l'expérience montre que cette plage de dimensions est trop large. On a en effet constaté que, pour une assimilation correcte, les particules ou plutôt les microparticules devaient être de dimension inférieure à 50 µm et de préférence encore de l'ordre de 10 µm.

C'est ainsi que la présente invention se rapporte à un médicament sous forme de granules à base de fénofibrate, chaque granule comportant un noyau neutre, une couche à base de fénofibrate et une couche de protection, caractérisé en ce que, dans la couche à base de fénofibrate, celui-ci est présent sous forme de microparticules cristallines de dimension inférieure à 50 µm, avantageusement inférieure à 30 µm, et de préférence inférieure à 10 µm les microparticules étant maintenues par un liant soluble notamment dans l'eau.

D'autres précisions sur ce médicament sont données dans les sous-revendications 2 à 5. Si celles-ci visent à lui conférer une forme optimale pour une administration et un dosage aussi faibles que possible selon des principes connus, on retiendra la taille des microparticules de fénofibrate comme l'enseignement essentiel de l'invention.

La présente invention vise donc un procédé de préparation garantissant que les microparticules de fénofibrate dans les granules du médicament obtenu ne dépassent pas une dimension de 50 µm.

Selon la présente invention, un tel procédé est caractérisé en ce qu'il comprend l'étape d'humidifier le noyau avec une couche extérieure humide et collante, puis l'étape de projeter sur le noyau humidifié des microparticules de fénofibrate de dimension inférieure à 50 µm de sorte qu'elles y restent collées, et enfin l'étape de sécher le tout avant que la couche humide ne dissolve les microparticules de fénofibrate, ces trois étapes étant le cas échéant répétées dans l'ordre jusqu'à ce qu'une quantité suffisante de fénofibrate soit fixée sur le noyau.

Avantageusement, la couche humide est alors formée par une solution dans un alcool pharmaceutiquement acceptable ou par une suspension dans l'eau du liant.

Bien que les différentes étapes de ce procédé soient globalement connues, par exemple la demande allemande citée plus haut les décrit, on ne les avait jusqu'à présent pas appliquées au fénofibrate ni à une taille maximale de microparticules aussi réduite.

On, cette application ne saurait être tenue pour allant de soi. Selon l'invention, l'obtention d'une première couche ne contenant que des microparticules de dimension inférieure à 50 microns, oblige notamment à sécher les granules très rapidement après la projection du fénofibrate sous peine de les voir se dissoudre. L'enseignement allemand ne présente pas quant à lui la rapidité du séchage comme un impératif technique. Ce dernier fait par conséquent partie de l'aspect inventif du présent procédé en relation avec les caractéristiques du médicament qu'il permet de préparer.

On va décrire maintenant, à titre d'exemple, la fabrication d'une forme de réalisation du médicament selon la présente invention.

On prépare des grains neutres pour former les noyaux ou âmes neutres, de façon classique. Par exemple, chaque grain peut être un cristal de saccharose de 0,3 mm de diamètre. Sur ces cristaux, on pulvérise une suspension d'amidon de maïs à 27 5 en poids dans du sirop de sucre à chaud (préparé par exemple en dissolvant 73 kg de sucre dans 32 kg d'eau : 27 kg d'amidon, 73 kg de sucre, 32 kg d'eau). Le sirop est projeté à 50°C dans une turbine elle-même chauffée à 50°C. La quantité projetée est ajustée pour que, quand le diamètre de chaque grain passe de 0,3 à 0,6 mm, le grain ait une teneur en amidon de 25 % environ en poids, pour 75 % environ de saccharose, une fois l'eau du sirop évaporée.

Ensuite, on met les noyaux neutres en rotation dans une turbine, et on les mouille avec une solution alcoolique à 12,5 % en poids d'un polymère métacrylique (alcool à 95°). Les grains deviennent humides et collants. On projette alors une poudre de fénofibrate, obtenue en broyant des cristaux jusqu'à obtention de microparticules

Les microparticules doivent avoir des dimensions inférieures à 50 µm, et de préférence inférieures à 30 µm. Avantageusement, une partie des particules a une dimension inférieure à 10 microns. Une poudre typique présente la distribution de dimensions suivantes :

100 % < 30 µm
99,5 % < 20 µm
98 % < 10 µm
88 % < 5 µm

On sèche aussitôt très rapidement pour ne pas laisser le temps à l'alcool de dissoudre le fénofibrate (courant d'air dans la turbine). On évite la destruction de la structure micronisée qui présente une surface considérable favorisant l'absorption. On ne peut déposer ainsi qu'une seule épaisseur de microparticules, fixées par adhérence sur le grain collant. L'opération mouillage - projection - séchage peut être faite en 1 ou 2 minutes environ. On recommence ces opérations de mouillage et de projection de microparticules jusqu'à ce que la totalité de la poudre soit incorporée.

Enfin, on procède à un enrobage de protection par exemple par une mince couche de polymère métacrylique, représentant 1 % en poids environ de chaque granule.

Les granules ainsi obtenus sont répartis en gelules, avec un dosage de 250 mg de fénofibrate par gelule.

La structure de la couche de fénofibrate est analogue à celle d'une éponge, dont les pores contiennent des microparticules de fénofibrate. L'éponge est constituée par un liant soluble dans un milieu aqueux : métacrylate ou polyvinylpyrolidone. Une fois le liant dissous, les microparticules de fénofibraate sont libérées et peuvent présenter la totalité de leurs surfaces pour le processus d'absorption dans les milieux acqueux intestinaux.

Un exemple de formulation est le suivant :
- fénofibrate : 400 kg
- grains neutres : 110 kg (sucre et/ou amidon)
- polyvinylpyrolidone et/ou métacrylate : 20 kg

Sur les 20 derniers kg, environ 5 kg sont utilisés pour former l'enveloppe de protection (environ 1 % en poids du total), le reste, environ 15 kg, est utilisé pour lier les microparticules de fénofibrate, avec utilisation temporaire d'alcool comme solvant.

La quantité de liant est déterminée pour que l'on obtienne au moins 65 % de fénofibrate libéré en une heure, dans un milieu liquide à base aqueuse.

Cette proportion peut être mesurée de la façon suivante : le contenu d'une gélule est placé dans un flacon contenant 25 ml d'un milieu de pH 1,5. Le flacon est soumis à une agitation de 30 tours/minute à 37 °C. Après une heure d'agitation, le pourcentage de fénofibrate libéré de la forme galénique selon l'invention est supérieur à 65 %.

Composition du milieu :
- 118 ml d'acide chlorhydrique normal
- 84 ml de solution d'hydroxyde de sodium normal
- eau distillée : quantité suffisante pour obtenir 1000,0 ml de milieu.

Le pH du milieu est compris entre 1,45 et 1,55.

Les médicaments de l'invention ont en outre montré une diminution de la variabilité des taux sanguins inter et intra patient (sur le même patient, et entre des patients distincts).

**Revendications**

1. Médicament sous forme de granules à base de fénofibrate, chaque granule comportant un noyau neutre, une couche à base de fénofibrate et une couche de protection, caractérisé en ce que, dans la couche à base de fénofibrate, celui-ci est présent sous forme de microparticules cristallines de dimension inférieure à 50 µm, avantageusement inférieure à 30 µm, et de préférence inférieure à 10 µm, les microparticules étant maintenues par un liant soluble notamment dans l'eau.

2. Médicament selon la revendication 1, caractérisé en ce que le liant est choisi parmi les polymères métacryliques, le polyvinylpyrolidone et leurs mélanges, des dérivés appropiés de la cellulose et les polyéthylènes glycols.

3. Médicament selon une des revendications 1 ou 2, caractérisé en ce que le noyau neutre a un diamètre d'environ 0,3 à 0,6 mm et est constitué d'un corps choisi parmi le glucose, saccharose, lactose et les autres sucres, et l'amidon, notamment l'amidon de maïs, et leurs mélanges.

4. Médicament selon une des revendications précédentes, caractérisé en ce que la couche de protection représente environ 1% en poids dans chaque granule, et est formée d'une matière choisi parmi les polymères métacryliques, le polyvinylpyrolidone et leurs mélanges, les dérivés de la cellulose et les polyéthylènes glycols.

5. Médicament selon une des revendications précédentes, caractérisé en ce que la quantité de liant est telle que la quantité de fénofibrate libéré en une heure dans un liquide à base aqueuse n'est pas inférieure à 65%.

6. Procédé de préparation d'un médicament sous forme de granules, selon une des revendications 1 à 5, caractérisé en ce qu'il comprend l'étape d'humidifier le noyau avec une couche extérieure humide et collante, puis l'étape de projeter sur le noyau humidifié des microparticules de fénofibrate de dimension inférieure à 50 µm de sorte qu'elles y restent collées, et enfin l'étape de sécher le tout avant que la couche humide ne dissolve les microparticules de fénofibrate, ces trois étapes étant le cas échéant répétées dans l'ordre jusqu'à ce qu'une quantité suffisante de fénofibrate soit fixée sur le noyau.

7. Procédé selon la revendication 6, caractérisé en ce que la couche humide est formée par une solution dans un alcool pharmaceutiquement acceptable ou par une suspension dans l'eau du liant.

## Claims

1. Medicine in the form of granules based on fenofibrate, each granule comprising an inert core, a layer based on fenofibrate and a protective layer, characterized in that, in the layer based on fenofibrate, the latter is present in the form of crystalline microparticles of dimensions not greater than 50 µm, advantageously not greater than 30 µm, and preferentially not greater than 10 µm, said microparticles being maintained by a binder more particularly soluble in water.

2. Medicine according to claim 1, characterized in that said binder is selected among the methacrylic polymers, the polyvinylpyrolidone and mixtures thereof, appropriate cellulose derivatives and polyethylene glycols.

3. Medicine according claim 1 or claim 2, characterized in that said inert core has a diameter of about 0,3 to 0,6 mm and is constituted by a substance selected from the glucose, the sucrose, the lactose and the other sugars, and starch, more particularly maize starch, and mixtures thereof.

4. Medicine according one of the preceding claims, characterized in that the protective layer represents about 1 % by weight of each granule, and is formed of a substance selected from the methacrylic polymers, the polyvinylpyrolidone and mixtures thereof,the cellulose derivatives and the polyethylene glycols.

5. Medicine according one of the preceding claims, characterized in that the quantity of binder is such that the quantity of fenofibrate liberated in one hour in an aqueous liquid is not less than 65 %.

6. Method for preparing a medicine in the form of granules according one of claims 1 to 5, characterized in that it comprises the step of damping said core with an external damp and sticky layer, and the next step of projecting on the dampened core fenofibrate microparticles, the size of which are not greater than 50 µm so that they adhere thereon, and finally the step of drying the all before the damping layer dissolves the fenofibrate microparticles, these three steps being eventually sequentially repeated until a suffisant quantity of fenofibrate is fixed on the core.

7. Method according to claim 6, characterized in that the damping layer is constituted by a solution in a pharmaceutically acceptable alcohol or by a suspension in the binder water.

## Patentansprüche

1. Medikament in der Form von Körnchen auf Fenofibratbasis, wobei jedes Körnchen einen neutralen Kern, eine Schicht auf der Basis von Fenofibrat und eine Schutzschicht aufweist, dadurch gekennzeichnet, daß in der Schicht auf der Basis von Fenofibrat dieses in der Form von kristallinen Mikroteilchen vorhanden ist, die kleiner als 50 µm, vorteilhafterweise kleiner als 30 µm und vorzugsweise kleiner als 10 µm sind, wobei die Mikroteilchen durch ein insbesondere in Wasser lösliches Bindemittel gehalten werden.

**2.** Medikament nach Anspruch 1, dadurch gekennzeichnet, daß das Bindemittel aus den Methacrylpolymeren, dem Polyvinylpyrrolidon und ihren Mischungen, geeigneten Derivaten der Cellulose und den Polyäthylenglykolen ausgewählt wird.

**3.** Medikament nach einem der Ansprüche 1 oder 2, dadurch gekennzeichnet, daß der neutrale Kern einen Durchmesser von etwa 0,3 bis 0,6 mm hat und aus einem Körper besteht, der aus Glucose, Saccharose, Lactose und den anderen Zuckern, und Stärke, insbesondere Maisstärke, und ihren Mischungen ausgewählt wird.

**4.** Medikament nach einem der vorhergehenden Ansprüche, dadurch gekennzeichnet, daß die Schutzschicht etwa 1 Gew.-% in jedem Körnchen darstellt und aus einer Substanz besteht, die aus den Methacrylpolymeren, dem Polyvinylpyrrolidon und ihren Mischungen, den Derivaten der Cellulose und den Polyäthylenglykolen ausgewählt wird.

**5.** Medikament nach einem der vorhergehenden Ansprüche, dadurch gekennzeichnet, daß die Menge an Bindemittel so gewählt ist, daß die Menge an in einer Stunde in einer Flüssigkeit auf Wasserbasis freigesetztem Fenofibrat nicht geringer als 65% ist.

**6.** Verfahren zur Herstellung eines Medikaments in Körnchenform, nach einem der Ansprüche 1 bis 5, dadurch gekennzeichnet, daß es eine Stufe, in der der Kern mit einer äußeren feuchten und klebenden Schicht angefeuchtet wird, dann eine Stufe, in der Mikrofibrat-Mikroteilchen einer Größe kleiner als 50 µm auf den angefeuchteten Kern aufgebracht werden, derart, daß sie darauf kleben bleiben, und schließlich eine Stufe aufweist, in der das Ganze getrocknet wird, bevor die feuchte Schicht die Fenofibrat-Mikroteilchen auflöst, wobei diese drei Stufen gegebenenfalls in dieser Reihenfolge wiederholt werden, bis eine ausreichende Menge von Fenofibrat auf dem Kern befestigt ist.

**7.** Verfahren nach Anspruch 6, dadurch gekennzeichnet, daß die feuchte Schicht aus einer Lösung in einem pharmazeutisch annehmbaren Alkohol oder aus einer Suspension des Bindemittels in Wasser besteht.